(19) Europäisches Patentamt
European Patent Office
Office européen des brevets

(11) Publication number: **0 337 600 A2**

(12) **EUROPEAN PATENT APPLICATION**

(21) Application number: **89301817.6**

(51) Int. Cl.4: **C07C 127/22 , A01N 47/34**

(22) Date of filing: **24.02.89**

(30) Priority: **26.02.88 JP 44863/88**
**10.05.88 JP 114467/88**
**01.07.88 JP 165555/88**
**26.08.88 JP 212704/88**

(43) Date of publication of application:
**18.10.89 Bulletin 89/42**

(84) Designated Contracting States:
**BE CH DE ES FR GB IT LI NL**

(71) Applicant: **SUMITOMO CHEMICAL COMPANY, LIMITED**
**Kitahama 4-chome 5-33**
**Chuo-ku Osaka 541(JP)**

(72) Inventor: **Sakamoto, Noriyasu**
**Sumitomokagaku Mefuryo, 14-7**
**Mefu-2-chome Takarazuka-shi(JP)**
Inventor: **Ohsumi, Tadashi**
**7-10-202, Kawazoecho**
**Nishinomiya-shi(JP)**
Inventor: **Mori, Tatsuya**
**Sumitomokagaku Mefuryo, 14-7**
**Mefu-2-chome Takarazuka-shi(JP)**
Inventor: **Yano, Toshihiko**
**15-10-103, Kusunokicho**
**Ashiya-shi(JP)**
Inventor: **Fujimoto, Izumi**
**9-17-374, Minoo-4-chome**
**Minoo-shi(JP)**
Inventor: **Takada, Yoji**
**10-4-454, Sonehigashinocho-2-chome**
**Toyonaka-shi(JP)**

(74) Representative: **Harrison, David Christopher et al**
**MEWBURN ELLIS & CO 2/3 Cursitor Street**
**London EC4A 1BQ(GB)**

(54) **Benzoylurea derivative, process for producing the same, insecticide containing the same, reaction intermediate of the same and process for producing the intermediate.**

(57) A benzoylurea derivative represented by the following formula,

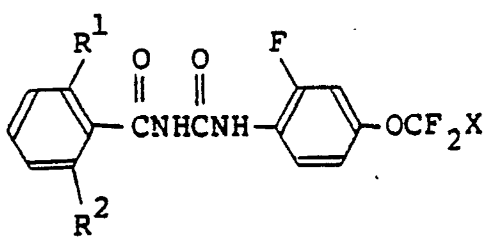

(I)

wherein $R^1$ is a fluorine or chlorine atom, $R^2$ is a fluorine atom when $R^1$ is a fluorine atom, and $R^2$ is a hydrogen atom when $R^1$ is a chlorine atom, and X is $CF_2Br$, $CCl_2H$, $CBr_2H$, CFHCl, $CFHCF_3$, $CFHOCF_3$, $CFHOC_2F_5$ or $CFHOC_3F_7$-n, is a useful insecticide or ovicide and can be formulated it into a composition for such use.

An intermediate from which the benzoylurea (I) can be prepared is an aniline of the formula

EP 0 337 600 A2

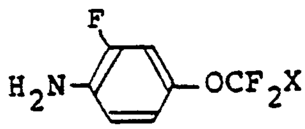

(III)

wherein X is as defined above.

# BENZOYLUREA DERIVATIVE, PROCESS FOR PRODUCING THE SAME, INSECTICIDE CONTAINING THE SAME, REACTION INTERMEDIATE OF THE SAME AND PROCESS FOR PRODUCING THE INTERMEDIATE

The present invention relates to a novel benzoylurea derivative represented by the following formula (I) [hereinafter referred to as present compound(s)], a process for producing the same, insecticides containing the same as an active ingredient, a reaction intermediate of the same and a process for producing the intermediate:

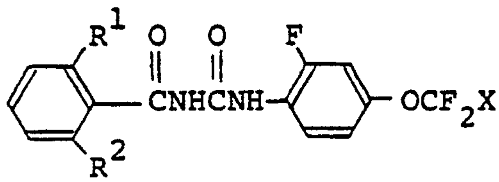
(I)

wherein $R^1$ is a fluorine or chlorine atom, $R^2$ is a fluorine atom when $R^1$ is a fluorine atom, and $R^2$ is a hydrogen atom when $R^1$ is a chlorine atom, and X is $CF_2Br$, $CCl_2H$, $CBr_2H$, CFHCl, $CFHCF_3$, $CFHOCF_3$, $CFHOC_2F_5$ or $CFHOC_3F_7$-n.

Hitherto, some benzoylurea compounds have been known to have an insecticidal activity (US Patent Nos. 3,933,908, 4,139,636 and 4,457,943, EP Nos. 71,279A and 235,089A, JP-A-59-106,454 and EP Nos. 226,642A and 203,618A). Some of them are already on the market.

These compounds, however, are poor in the efficacy or have a demerit in the manufacturing cost, so that they are not always said to be satisfactory.

In view of such situation, the present inventors have extensively studied to develop a benzoylurea compound having a more superior insecticidal activity. As a result, the present inventors have found that a benzoylurea derivative represented by the foregoing formula (I) has a high insecticidal activity, that it exhibits a particularly high lethal activity against larvae, nymphs and eggs of insect pests, and also that it can be produced more economically than before. The present inventors thus attained to the present invention.

Specific examples of the insect pests and eggs against which the present compounds are particularly efficacious are the larvae and eggs of Lepidoptera such as diamond-back moth (Plutella xylostella), rice stem borer (Chilo suppressalis), armyworms and cutworms, Plusiid moth (Plusiinae), small white butterfly (Pieris rapae crucivora), casemaking clothes moth (Tinea pellionella), webbing clothes moth (Tineola bisselliella), etc.; the larvae and eggs of Diptera such as house mosquitoes (Culex spp.), Anopheline mosquitoes (Anopheles spp.), Aedes mosquitoes (Aedes spp.), chironomid midges (Chironomidae), houseflies (Muscidae), blow flies (Calliphoridae), flesh flies (Scarcophagidae), tabanid flies (Tabanidae), blackflies (Simuliidae), etc.; the nymphs and eggs of Dictyoptera such as German cockroach (Blattella germanica,) smokybrown cockroach (Perilaneta fuliginosa), brown cockroach (Perilaneta brunnea), American cockroach (Perilaneta americana), etc.; and the larvae and eggs of Coleoptera and Hymenoptera; etc.

Also, the present compounds have low toxicity to warm-blooded animals so that they can be orally administered to domestic animals such as cattle, pigs, horses, sheep, goats, chickens, etc. mixed in the feeds for the animals. As a result, the present compounds are excreted undecomposed from the animals, so that the larvae and eggs of insects living in the excrements of the domestic animals [e.g. housefly (Musca domestica), false stablefly (Muscina stabulans), little housefly (Fannia canicularis), blow flies (Calliphoridae), flesh flies (Sarcophagidae), sepsid flies ( Sepsidae)] can be exterminated.

The benzoylurea derivatives according to the present invention are represented by the formula,

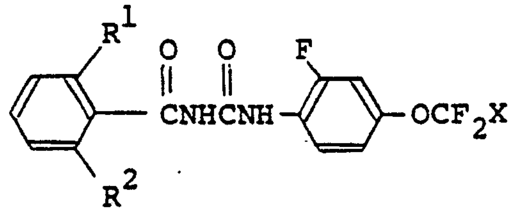

wherein $R^1$ is a fluorine or chlorine atom, $R^2$ is a fluorine atom when $R^1$ is a fluorine atom, and $R^2$ is a hydrogen atom when $R^1$ is a chlorine atom, and X is $CF_2Br$, $CCl_2H$, $CBr_2H$, CFHCl, $CFHCF_3$, $CFHOCF_3$, $CFHOC_2F_5$ or $CFHOC_3F_7$-n.

Of these benzoylurea derivatives, preferred are those in which $R^1$ and $R^2$ are the same meanings as described above, and X is CFHCl, $CFHCF_3$, $CFHOCF_3$ or $CFHOC_3F_7$-n. More preferred are those in which both $R^1$ and $R^2$ are a fluorine atom, and X is CFHCl, CFHCl, $CFHCF_3$, $CFHOCF_3$ or $CFHOC_3F_7$-n.

The present compounds represented by the formula (I) can be produced by the following processes.

Process A:

A process comprising allowing to react a benzoylisocyanate compound represented by the formula (II),

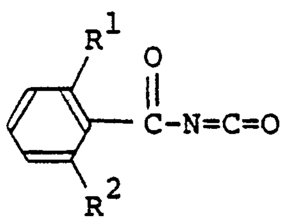

(II)

wherein $R^1$ and $R^2$ are the same meanings as defined above, with an aniline compound represented by the formula (III),

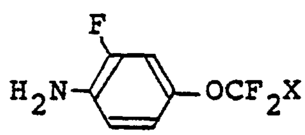

(III)

wherein X is the same meaning as defined above.

Process B:

A process comprising allowing to react a benzamide compound represented by the formula (IV),

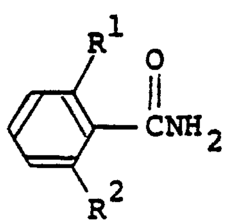

(IV)

wherein $R^1$ and $R^2$ are the same meanings as defined above, with an isocyanate compound represented by the formula (V),

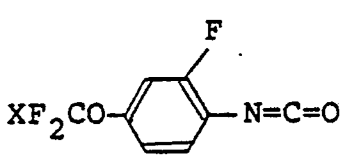

(V

wherein X is the same meaning as defined above.

In the foregoing Processes A and B, the reaction in usually carried out in the presence of an inert solvent. The solvent usable includes for example aromatic hydrocarbons (e.g. benzene, toluene, xylene),

halogenated hydrocarbons (e.g. chlorobenzene, carbon tetrachloride, chloroform, methylene chloride, 1,2-dichloroethane), ethers (e.g. diethyl ether, tetrahydrofuran, dioxane), ketones (e.g. acetone, methyl ethyl ketone, methyl isobutyl ketone), dimethyl sulfoxide, dimethylformamide, nitromethane and mixtures thereof.

In Processes A and B, the reaction can generally be carried out under normal pressure, and the satisfactory results can usually be obtained in 10 minutes to 50 hours. The starting materials are generally used in the same molar amount, but one of the compounds may be used in excess. Specifically, the benzoylisocyanate compound (II) is used in a ratio of from 0.8 to 1.2 moles per mole of the aniline compound (III), and the isocyanate compound (V) is used in a ratio of from 0.8 to 1.2 moles per mole of the benzamide compound (IV).

In Processes A and B, the reaction temperature is not particularly limited. In Process A, the reaction temperature is in a range of generally from 0° to 80°C, usually from room temperature (ca. 20°C) to 60°C. In Process B, it is in a range of generally from room temperature to 160°C, usually from 80° to 130°C.

The present compounds thus obtained can be purified if necessary by means such as column chromatography, recrystallization, etc.

The present compounds (I) thus produced include the following:

N-2,6-difluorobenzoyl-N′-[2-fluoro-4-(2-chloro-1,1,2-trifluoroethoxy)phenyl]urea,
N-2-chlorobenzoyl-N′-[2-fluoro-4-(2-chloro-1,1,2-trifluoroethoxy)phenyl]urea,
N-2,6-difluorobenzoyl-N′-[2-fluoro-4-(2-bromo-1,1,2,2-tetrafluoroethoxy)phenyl]urea, N-2-chlorobenzoyl-N′-[2-fluoro-4-(2-bromo-1,1,2,2-tetrafluoroethoxy)phenyl]urea,
N-2,6-difluorobenzoyl-N′-[2-fluoro-4-(2,2-dibromo-1,1-difluoroethoxy)phenyl]urea,
N-2-chlorobenzoyl-N′-[2-fluoro-4-(2,2-dibromo-1,1-difluoroethoxy)phenyl]urea,
N-2,6-difluorobenzoyl-N′-[2-fluoro-4-[1,1,2- trifluoro-2-(trifluoromethoxy)ethoxy]phenyl]urea,
N-2-chlorobenzoyl-N′-[2-fluoro-4-[1,1,2-trifluoro-2-(trifluoromethoxy)ethoxy]phenyl]urea,
N-2,6-difluorobenzoyl-N′-[2-fluoro-4-(2,2-dichloro-1,1-difluoroethoxy)phenyl]urea,
N-2-chlorobenzoyl-N′-[2-fluoro-4-(2,2-dichloro-1,1-difluoroethoxy)phenyl]urea,
N-2,6-difluorobenzoyl-N′-[2-fluoro-4-[1,1,2-trifluoro-2-(pentafluoroethoxy)ethoxy]phenyl]urea,
N-2-chlorobenzoyl-N′-[2-fluoro-4-[1,1,2-trifluoro-2-(pentafluoroethoxy)ethoxy]phenyl]urea,
N-2,6-difluorobenzoyl-N′-[2-fluoro-4-[1,1,2-trifluoro-2-(heptafluoropropoxy)ethoxy]phenyl]urea,
N-2-chlorobenzoyl-N′-[2-fluoro-4-[1,1,2-trifluoro-2-(heptafluoropropoxy)ethoxy]phenyl]urea,
N-2,6-difluorobenzoyl-N′-[2-fluoro-4-(1,1,2,3,3,3-hexafluoropropoxy)phenyl]urea, and N-2-chlorobenzoyl-N′-[2-fluoro-4-(1,1,2,3,3,3-hexafluoropropoxy)phenyl]urea

Production examples for the present compounds will be shown below.

Production Example 1

0.31 Gram of 2-fluoro-4-(2-chloro-1,1,2-trifluoroethoxy)aniline was dissolved in 5 ml of toluene, and a solution of 0.23 g of 2,6-difluorobenzoylisocyanate in 3 ml of toluene was added dropwise to the resulting solution with stirring and ice-cooling. After completion of the addition, the reaction solution was stirred overnight at room temperature. Thereafter, 5 ml of n-hexane was added thereto. The formed crystals were collected by filtration and dried to obtain 0.39 g of N-2,6-difluorobenzoyl-N′-[2-fluoro-4-(2-chloro-1,1,2-trifluoroethoxy)phenyl]urea as white crystals.

| | |
|---|---|
| Yield | 72% |
| m.p. | 178.4°C |

Production Example 2

0.30 Gram of 2-fluoro-4-(2-bromo-1,1,2,2-tetrafluoroethoxy) aniline was added to 33 ml of a 5% phosgene-toluene solution, and the resulting mixture was heated under reflux for 3 hours. After concentrating the reaction solution, the residue obtained was dissolved in 20 ml of xylene and 0.15 g of 2,6-difluorobenzamide was added thereto. The mixture was stirred under reflux for 24 hours. The reaction solution was cooled and concentrated to obtain a crude product. The crude product was subjected to chromatography on silica gel to obtain 0.41 g of N-2,6-difluorobenzoyl-N′-[2-fluoro-4-(2-bromo-1,1,2,2-tetrafluoroethoxy)phenyl]urea as white crystals.

| | |
|---|---|
| Yield | 85% |
| m.p. | 186.7 °C |

Production Example 3

0.26 Gram of 2-fluoro-4-[1,1,2-trifluoro-2-(trifluoromethoxy)ethoxy]aniline was dissolved in 6 ml of toluene, and to the resulting solution was added dropwise a solution of 0.16 g of 2,6-difluorobenzoyl isocyanate in 4 ml of toluene with stirring and ice-cooling. After completion of the addition, the reaction solution was stirred overnight at room temperature, and 5 ml of n-hexane were added thereto. The precipitated crystals were filtered off and dried to obtain 0.26 g of N-2,6-difluorobenzoyl-N′-[2-fluoro-4-(1,1,2-trilfluoro-2-(trifluoromethoxy)ethoxy)phenyl]urea as white crystals.

| | |
|---|---|
| Yield: | 63% |
| m.p.: | 125.6 °C |

Examples of the present compound obtained by these processes are shown in Table 1.

Table 1

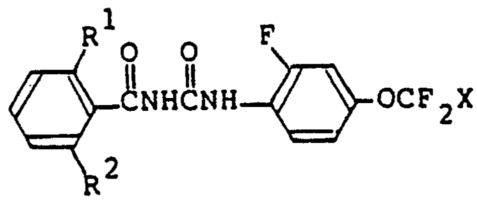

| Compound No. | $R^1$ | $R^2$ | X | Physical constant m.p. (°C) |
|---|---|---|---|---|
| (1) | F | F | $-CF_2Br$ | 186.7 |
| (2) | F | F | $-CCl_2H$ | 185.7 |
| (3) | Cl | H | $-CCl_2H$ | 160.8 |
| (4) | F | F | $-CBr_2H$ | 189.2 |
| (5) | F | F | -CFClH | 178.4 |
| (6) | Cl | H | -CFClH | 159.8 |
| (7) | F | F | $-CFHCF_3$ | 172.2 |
| (8) | Cl | H | $-CFHCF_3$ | 147.6 |
| (9) | F | F | $-CFHOC_3F_7$-n | 112.5 |
| (10) | Cl | H | $-CFHOC_3F_7$-n | 117.1 |
| (11) | F | F | $-CFHOCF_3$ | 125.6 |
| (12) | Cl | H | $-CFHOCF_3$ | 128.1 |

In the processes of the present invention, the benzoylisocyanate compound represented by the formula (II) and the benzamide compound represented by the formula (IV), both of which are starting materials, are known compounds. The aniline compound represented by the formula (III) is a novel compound and can be produced, for example, by the processes shown below.

Process I:

A process for producing an aniline compound (III′) comprising allowing to react 3-fluoro-4-amino-phenol with an olefin compound (VI) in the presence of a base.

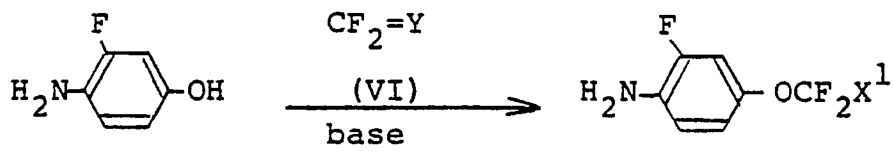


(III')

wherein $X^1$ is $CCl_2H$, $CBr_2H$, CFHCl, $CFHOCF_3$, $CFHOC_2F_5$ or $CFHOC_3F_7$-n and Y is $CCl_2$, $CBr_2$, CFCl, $CFOCF_3$, $CFOC_2F_5$ or $CFOC_3F_7$-n.

The base used in this reaction includes for example caustic alkalis (e.g. caustic potash), alkali carbonates (e.g. potassium carbonate), alkali metals (e.g. metallic sodium), alkali metal hydrides (e.g. sodium hydride), etc. However, caustic potash is preferably used when $X^1$ is not $CFHOCF_3$, $CFHOC_2F_5$ or $CFHOC_3F_7$-n, and sodium hydride is preferably used when $X^1$ is $CFHOCF_3$, $CFHOC_2F_5$ or $CFHOC_3F_7$-n.

The reaction of the present invention is usually carried out in an inert solvent, and the solvent includes for example dimethylformamide, mixtures of dimethylformamide with other inert solvents (e.g. toluene, acetonitrile, dioxane), etc. Preferably, dimethylformamide is used. The reaction temperature is usually 0° to 150°C, but it is preferably 70° to 100°C when $X^1$ is not $CFHOCF_3$, $CFHOC_2F_5$ or $CFHOC_3F_7$-n and 0°C to room temperature when $X^1$ is $CFHOCF_3$, $CFHOC_2F_5$ or $CFHOC_3F_7$-n. In carrying out the reaction of the present invention, at least one mole of the olefin compound (VI) is used per mole of 3-fluoro-4-aminophenol. The amount of the base used is from a catalytic amount to 3 moles per mole of 3-fluoro-4-aminophenol. Preferably, the base is used in a catalytic amount or so when $X^1$ is not $CFHOCF_3$, $CFHOC_2F_5$ or $CFHOC_3F_7$-n and about 1 mole when $X^1$ is $CFHOCF_3$, $CFHOC_2F_5$ or $CFHOC_3F_7$-n.

After completion of the reaction, the reaction solution is poured into water, treated as usual by extraction with organic solvents, concentration, etc. and if necessary, purified by means such as chromatography, distillation, recrystallization, etc.

Process II:

A method for producing an aniline compound (III) comprising allowing to react 3-fluoro-4-nitrophenol with an olefin compound (VI), hexafluoropropylene or 1,2-dibromotetrafluoroethane in the presence of a base followed by reducing the resulting nitrobenzene compound (VII) with iron in the presence of an acid or catalytically reducing the compound (VII) with hydrogen in the presence of platinum dioxide.

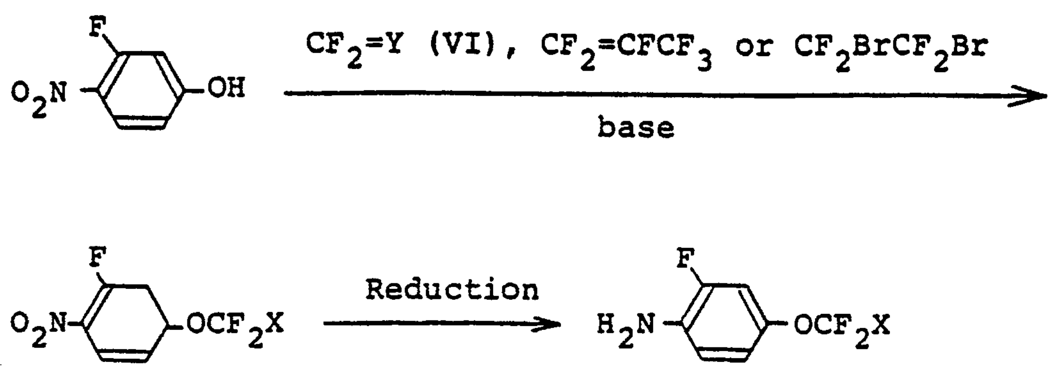


(VII) (III)

wherein X and Y are the same meanings as defined above.

In this process, the reaction of 3-fluoro-4-nitrophenol with an olefin compound (VI) or hexafluoropropylene is carried out according to the reaction conditions of Process I. In the case 3-fluoro-4-nitrophenol is allowed to react with 1,2-dibromotetrafluoroethane, the base usually used includes alkali metal hydrides (e.g. sodium hydride, potassium hydride), preferably sodium hydride. The reaction is usually carried out in an inert solvent. As the solvent, dimethylformamide, dimethyl sulfoxide, tetrahydrofuran, etc.

are usually used, for example, and dimethylformamide is preferably used. The reaction temperature is usually from 0° to 100° C, preferably from 0° to 30° C. The base is used in 0.5 to 3 moles, preferably about 1 mole per mole of 3-fluoro-4-nitrophenol. And 1,2-dibromotetrafluoroethane is used in at least one mole per mole of 3-fluoro-4-nitrophenol.

After completion of the reaction, the reaction solution is treated as usual by extraction with organic solvents, concentration, etc. and if necessary, purified by means such as chromatography, distillation, recrystallization, etc.

The reduction of the compound (VII) to the aniline compound (III) is carried out according to the conventional technique such as for example reduction with iron in the presence of an acid, catalytic reduction with hydrogen in the presence of platinum dioxide, etc.

After completion of the reduction, the resulting solution is treated as follows: In the case of reduction with iron, the resulting solution is poured into an aqueous sodium hydrogencarbonate solution and treated as usual by extraction with organic solvents, concentration, etc.; and in the case of catalytic reduction, the metallic catalyst is removed by filtration and the filtrate is treated as usual by concentration, etc. In any case, the resulting aniline compound (III) is purified, if necessary, by means such as chromatography, distillation, recrystallization, etc.

Process III:

A process for producing an aniline compound (III″) comprising allowing to react 4-acetylamino-3-fluorophenol with an olefin compound (VI) or 1,2-dibromotetrafluoroethane in the presence of a base to obtain an acetanilide compound (VIII) followed by hydrolyzing the acetylamino group of the compound (VIII) by a usual hydrolysis technique.

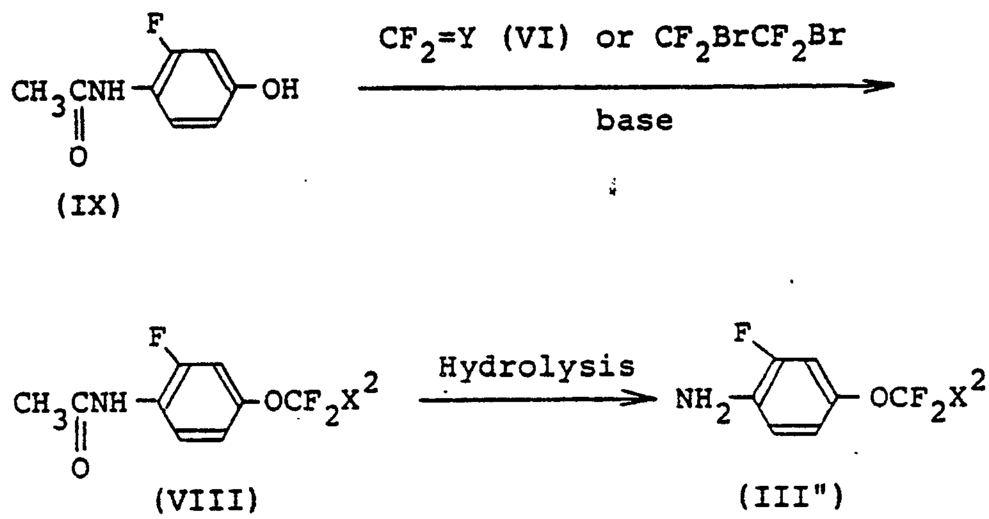


(IX)

(VIII)

(III")

wherein $X^2$ is $CCl_2H$, $CBr_2H$, $CFHCl$, $CF_2Br$, $CFHOCF_3$, $CFHOC_2F_5$ or $CFHOC_3F_7$-n and Y is the same meaning as defined above.

In this process, the reaction of 4-acetylamino-3-fluorophenol (IX) with an olefin compound (VI) is carried out according to the reaction conditions of Process I. The reaction of the compound (IX) with 1,2-dibromotetrafluoroethane is carried out according to the reaction conditions of Process II.

The hydrolysis of the acetanilide compound (VIII) to the aniline compound (III″) is carried out according to a conventional technique and the conditions are not particularly limited. For example, it is carried out in a solvent in the presence of an acid or base, and the conditions can be determined according to a conventional technique. The acid used includes mineral acids (e.g. hydrochloric acid, sulfuric acid), and the base used includes caustic alkalis (e.g. caustic potash, caustic soda), alkali carbonates (e.g. potassium carbonate, sodium carbonate), etc. The solvent, includes alcohols (e.g. methanol, ethanol, ethylene glycol), water and a mixture thereof.

The reaction temperture is usually from room temperature to 200° C, and the amount used of the acid or base is from 1 to 10 moles per mole of the acetanilide compound (VIII).

After completion of the reaction, the reaction solution is almost neutralized, treated as usual by

extraction with organic solvents, concentration, etc. and if necessary, purified by means such as chromatography, distillation, recrystallization, etc.

Subsequently, the aniline compound (III) can be converted to an isocyanate compound represented by the formula (V) by allowing to react the aniline compound as usual with phosgene. The reaction is usually carried out under the following conditions.

The amount of phosgene used in this reaction is usually from 1 to 5 moles based on 1 mole of the aniline compound (III). In this reaction, an inert solvent is usually used, and such solvent includes for example hydrocarbons (e.g. hexane, heptane, benzene, toluene), halogenated hydrocarbons (e.g. dichloromethane, chloroform, 1,2-dichloroethane, chlorobenzene) and a mixture of two or more of them. Usually, this reaction well proceeds at a temperature of from room temperature to the boiling point of the solvent. The isocyanate compound (V) thus obtained can easily be purified by distillation, etc. if necessary.

The novel intermediate of the present invention thus obtained include the following:

Aniline compound (III):

2-Fluoro-4-(2-bromo-1,1,2,2-tetrafluoroethoxy)aniline,
2-Fluoro-4-(1,1-difluoro-2,2-dichloroethoxy)aniline,
2-Fluoro-4-(1,1-difluoro-2,2-dibromoethoxy)aniline,
2-Fluoro-4-(2-chloro-1,1,2-trifluoroethoxy)aniline,
2-Fluoro-4-(1,1,2,3,3,3-hexafluoropropoxy)aniline,
2-Fluoro-4-[1,1,2-trifluoro-2-(trifluoromethoxy)ethoxy]aniline,
2-Fluoro-4-[1,1,2-trifluoro-2-(pentafluoroethoxy)ethoxy]aniline, and
2-Fluoro-4-[1,1,2-trifluoro-2-(heptafluoropropoxy)ethoxy]aniline;

Isocyanate compound (V):

2-Fluoro-4-(2-bromo-1,1,2,2-tetrafluoroethoxy)phenylisocyanate,
2-Fluoro-4-(1,1-difluoro-2,2-dichloroethoxy) phenylisocyanate
2-Fluoro-4-(1,1-difluoro-2,2-dibromoethoxy)phenylisocyanate,
2-Fluoro-4-(2-chloro-1,1,2-trifluoroethoxy)phenylisocyanate,
2-Fluoro-4-(1,1,2,3,3,3-hexafluoropropoxy)phenylisocyanate,
2-Fluoro-4-[1,1,2-trifluoro-2-(trifluoromethoxy)ethoxy]phenylisocyanate,
2-Fluoro-4-[1,1,2-trifluoro-2-(pentafluoroethoxy)ethoxy]phenylisocyanate, and
2-Fluoro-4-[1,1,2-trifluoro-2-(heptafluoropropoxy)ethoxy]phenylisocyanate;

Nitrobenzene compound (VII):

1-Nitro-2-fluoro-4-(2-bromo-1,1,2,2-tetrafluoroethoxy)benzene,
1-Nitro-2-fluoro-4-(1,1-difluoro-2,2-dichloroethoxy)benzene,
1-Nitro-2-fluoro-4-(1,1-difluoro-2,2-dibromoethoxy)benzene,
1-Nitro-2-fluoro-4-(2-chloro-1,1,2-trifluoroethoxy)benzene,
1-Nitro-2-fluoro-4-(1,1,2,3,3,3-hexafluoropropoxy)benzene,
1-Nitro-2-fluoro-4-[1,1,2-trifluoro-2-(trifluoromethoxy)ethoxy]benzene,
1-Nitro-2-fluoro-4-[1,1,2-trifluoro-2-(pentafluoroethoxy)ethoxy]benzene, and
1-Nitro-2-fluoro-4-[1,1,2-trifluoro-2-(heptafluoropropoxy)ethoxy]benzene; and

Acetanilide compound (VIII):

2-Fluoro-4-(2-bromo-1,1,2,2-tetrafluoroethoxyacetanilide,
2-Fluoro-4-(1,1-difluoro-2,2-dichloroethoxy)acetanilide,
2-Fluoro-4-(1,1-difluoro-2,2-dibromoethoxy)acetanilide,
2-Fluoro-4-(2-chloro-1,1,2-trifluoroethoxy)acetanilide,
2-Fluoro-4-[1,1,2-trifluoro-2-(trifluoromethoxy)ethoxy]acetanilide,
2-Fluoro-4-[1,1,2-trifluoro-2-(pentafluoroethoxy)ethoxy]acetanilide, and

2-Fluoro-4-[1,1,2-trifluoro-2-(heptafluoropropoxy)ethoxy]acetanilide Production examples for these intermediate compounds will be shown below.

Production Example 4

0.60 Gram of 3-fluoro-4-aminophenol, 0.05 g of potassium hydroxide and 10 ml of dimethylformamide were charged in a reactor and stirred for 20 minutes in an oil bath at a temperature of from 60° to 70° C. This solution was then intensely stirred for 2 hours at the same temperature under the stream of a chlorotrifluoroethylene gas in an excess amount relative to the phenol. The reaction solution was cooled, and water was added thereto. Then the solution was extracted with two 150-ml portions of diethyl ether. The ether layers were combined, washed with water, dried and concentrated to obtain a crude product. This crude product was subjected to chromatography on silica gel to obtain 0.67 g of 2-fluoro-4-(2-chloro-1,1,2-trifluoroethoxy)aniline.
Yield 58%
$^1$H-NMR ($CDCl_3$): δ (ppm)
3.68 (2H, br.), 6.16 (1H, dt. J = 4, 48Hz), 6.47-7.07 (3H, m.)

The intermediate aniline compounds thus obtained will be shown below:
2-Fluoro-4-(1,1-difluoro-2,2-dichloroethoxy)-aniline, $^1$H-NMR ($CDC1_3$): δ (ppm)
3.69 (2H, br.), 5.84 (1H, t. J = 5Hz), 6.50-7.10 (3H, m.); and
2-Fluoro-4-(1,1-difluoro-2,2-dibromoethoxy)-aniline, $^1$H-NMR ($CDC1_3$): δ (ppm)
3.51 (2H, br.), 5.76 (1H, t. J = 6Hz), 6.50-7.21 (3H, m.)

Production Example 5

0.52 Gram of 3-fluoro-4-aminophenol and 8 ml of dimethylformamide were charged in a reactor, and 0.18 g of sodium hydride (60% oily suspension) was slowly added with stirring and ice-cooling. After the evolution of a hydrogen gas had ceased, 1.32 g of perfluoro vinyl propyl ether ($CF_2 = CFOC_3F_7$-n) was slowly added. After stirring for 10 minutes with ice-cooling, the reaction solution was heated back to room temperature. After 10 minutes, the reaction solution was poured into water and extracted with two 150-ml portions of ethyl acetate. The organic layers were combined, washed with water, dried and concentrated to obtain a crude product. This crude product was subjected to chromatography on silica gel to obtain 1.05 g of 2-fluoro-4-[1,1,2-trifluoro-2-(heptafluoropropoxy)ethoxy]aniline.
Yield 65%
$^1$H-NMR ($CDCl_3$ ): δ (ppm)
3.70 (2H, br.), 5.97 (1H, dt. J = 54, 3Hz), 6.59-7.10 (3H, m.)

Production Example 6

2.0 Grams of 3-fluoro-4-nitrophenol were dissolved in 15 ml of dimethylformamide, and the resulting solution was intensely stirred for 15 minutes at about 60° C under the stream of a hexafluoropropene gas in a large excess amount relative to the phenol. After quickly adding 0.25 g of potassium hydroxide to the reaction solution, the solution was intensely stirred for 2 hours under the same condition. The reaction solution was then cooled, and water was added thereto. Then, the solution was extracted with two 100-ml portions of diethyl ether. The ether layers were combined, dried and concentrated to obtain a yellow oily product as a residue. The oily product was subjected to chromatography on silica gel to obtain 1.20 g of 2-fluoro-4-(1,1,2,3,3,3-hexafluoropropoxy)nitrobenzene.
Yield 31%

1.20 Grams of 2-fluoro-4-(1,1,2,3,3,3-hexafluoropropoxy)nitrobenzene, 0.05 g of platinum dioxide and 10 ml of ethyl acetate were charged in a reactor. After replacing the air in the reactor by hydrogen gas with stirring, the mixture were stirred for 2 hours at room temperature while introducing a hydrogen gas to the mixture. The reaction solution was filtered, and the filtrate was concentrated to obtain 1.0 g of 2-fluoro-4-(1,1,2,3,3,3-hexafluoropropoxy)-aniline.
Yield 92%
$n_D^{22.5}$ 1.4219

Production Example 7

0.52 Gram of 4-acetylamino-3-fluorophenol and 1.60 g of 1,2-dibromodifluoroethane were dissolved in 10 ml of dimethylformamide in a reactor. After cooling the solution to 0°C, 0.14 g of sodium hydride (60% oily suspension) was added in portions with stirring. After the addition, stirring was continued for 12 hours at room temperature. The reaction solution was poured into a 5% aqueous hydrochloric acid, weakly acidified and extracted with two 100-ml portions of diethyl ether. The ether layers were combined, dried and concentrated to obtain a yellow oily product as a residue. The oily product was subjected to chromatography on silica gel to obtain 0.42 g of 4-acetylamino-3-fluoro-1-(2-bromo-1,1,2,2-tetrafluoroethoxy)benzene.
Yield 43%
$^1$H-NMR ($CDCl_3$) : $\delta$ (ppm)
2.21 (3H, s.), 6.85-7.20 (2H), 7.60 (1H, br.), 8.30 (1H, t. J=9.0Hz)
$^{19}$F-NMR ($CDCl_3/CF_3CO_2H$) : $\delta$ (ppm)
-47 (1F, s.), -7.5 (2F, s.), 10 (2F, s.)

0.43 Gram of 4-acetylamino-3-fluoro-1-)2-bromo-1,1,2,2-tetrafluoroethoxy)benzene and 10 ml of 20% aqueous hydrochloric acid were charged in a reactor and stirred under reflux for 2 hours. After cooling the reaction solution, a 5% aqueous sodium hydrogencarbonate solution was added to make the reaction solution weakly alkaline. The reaction solution was then extracted with two 100-ml portions of diethyl ether. The ether layers were combined, dried and concentrated to obtain a yellow oily product as a residue. The oily product was subjected to chromatography on silica gel to obtain 0.30 g of 2-fluoro-4-)2-bromo-1,1,2,2,-tetrafluoroethoxy)aniline.
Yield 80%
$^{19}$F-NMR ($CDCl_3/CF_3CO_2H$) : $\delta$ (ppm)
-52 (1F, s.), -8 (2F, s.), 10 (2F, s.)

When the present compounds are used as an active ingredient for insecticides, they are usually formulated into oil sprays, emulsifiable concentrates, wettable powders, flowable concentrates, granules, dusts, aerosols, foggings, poisonus baits, etc. by mixing with solid carriers, liquid carriers, gaseous carriers or baits and if necessary, adding surface active agents and other auxiliaries for formulation.

These preparations usually contain the present compounds as an active ingredient in an amount of from 0.01 to 95% by weight.

The solid carriers used in the formulation include for example fine powders or granules of clays (e.g. kaolin clay, diatomaceous earth, synthetic hydrated silicon dioxide, bentonite, Fubasami clay, terra alba), talcs, ceramics, other inorganic minerals (e.g. sericite, quartz, sulfur, activated carbon, calcium carbonate, hydrated silica), chemical fertilizers (e.g. ammonium sulfate, ammonium phosphate, ammonium nitrate, urea, ammonium chloride), etc. The liquid carriers include for example water, alcohols (e.g. methanol, ethanol), ketones (e.g. acetone, methyl ethyl ketone), aromatic hydrocarbons (e.g. benzene, toluene, xylene, ethylbenzene, methylnaphthalene), aliphatic hydrocarbons (e.g. hexane, cyclohexane, kerosene, gas oil), esters (e.g. ethyl acetate, butyl acetate), nitriles (e.g. acetonitrile, isobutyronitrile), ethers (e.g. diisopropyl ether, dioxane), acid amides (e.g. N,N-dimethylformamide, N,N-dimethylacetamide), halogenated hydrocarbons (e.g. dichloromethane, trichloroethane, carbon tetrachloride), dimethyl sulfoxide, vegetable oils (e.g. soybean oil, cotton seed oil), etc. The gaseous carriers, i.e. a propellant, include for example freon gas, butane gas, LPG (liquefied petroleum gas), dimethyl ether, carbon dioxide gas, etc.

The surface active agents include for example alkyl sulfates, alkylsulfonates, alkylarylsulfonates, alkyl aryl ethers and their polyoxyethylenized products, polyethylene glycol ethers, polyhydric alcohol esters, sugar alcohol derivatives, etc.

The auxiliaries for formulation such as fixing agents, dispersing agents, etc. include for example casein, gelatin, polysaccharides (e.g. starch powder, gum arabic, cellulose derivatives, alginic acid), lignin derivatives, bentonite, saccharides, synthetic water-soluble high polymers (e.g. polyvinyl alcohol, polyvinyl pyrrolidone, polyacrylic acids), etc. The stabilizing agents include for example PAP (isopropyl acid phosphate), BHT (2,6-di-tert-butyl-4-methylphenol), BHA (a mixture of 2-tert-butyl-4-methoxyphenol and 3-tert-butyl-4-methoxyphenol), vegetable oils, mineral oils, surface active agents, fatty acids and their esters, etc.

The base of the poisonous baits includes for example components (e.g. grain powders, vegetable essential oils, saccharides, crystalline celluloses), antioxidants (e.g. dibutylhydroxytoluene, nordihydroguaiaretic acid), preservatives (e.g. dehydroacetic acid), attractants (e.g. cheese perfume, onion perfume, peanut oil), etc. Further, red pepper powders etc. also are included as an agent for preventing children from eating by mistake.

The preparations thus obtained are used as they are or diluted with water, etc. Further, they may be

used mixed with other insecticides, nematocides, acaricides, soil-pest controlling agents, fungicides, herbicides, plant growth regulators, synergists, fertilizers, soil improvers, feeds for animals, etc., or may be used simultaneously with these chemicals without mixing.

When the present compounds are used as agricultural insecticides, their dosage rate is usually from 0.5 to 500 g/10 ares. When the emulsifiable concentrates, wettable powders, flowable concentrates, etc. are used diluted with water, the application concentration of the active ingredient is 1 to 1000 ppm. The granules, dusts, etc. are used as they are without being diluted. When the present compounds are used as household and public hygienic insecticides, the emulsifiable concentrates, wettable powders, flowable concentrates, etc. are applied diluted with water to 1 to 1000 ppm, and the oil sprays, aerosols, foggings, poisonous baits, etc. are applied as they are.

Although any of these dosage rate and application concentration depends on the type of preparations, when, where and how these preparations are applied, the kind of pests, the degree of damage, etc., they may be increased or decreased independently of the ranges explained above.

Formulation examples will be shown. In the examples, parts are by weight.

Formulation Example 1

Ten parts of each of the present compounds (1) to (12), 14 parts of polyoxyethylene styrylphenyl ether, 6 parts of calcium dodecylbenzenesulfonate, 35 parts of xylene and 35 parts of dimethylformamide are well mixed to obtain an emulsifiable concentrate of each compound.

Formulation Example 2

Twenty parts of each of the present compounds (1), (3), (6), (7), (9) and (11), 10 parts of Fenitrothion (O,O-dimethyl O-4-nitro-m-tolyl phosphorothioate), 3 parts of calcium lignosulfonate, 2 parts of sodium lauryl sulfate and 65 parts of synthetic hydrated silicon dioxide are well pulverized and mixed to obtain a wettable powder of each compound.

Formulation Example 3

One part of each of the present compounds (1), (2), (3), (7), (10) and (12), 2 parts of Carbaryl (1-naphthyl methylcarbamate), 87 parts of kaolin clay and 10 parts of talc are well pulverized and mixed to obtain a dust of each compound.

Formulation Example 4

Five parts of each of the present compounds (1), (3), (4), (7), (9) and (11), 1 part of synthetic hydrated silicon dioxide, 2 parts of calcium lignosulfonate, 30 parts of bentonite and 62 parts of kaolin clay are well pulverized and mixed. The resulting mixture is well kneaded with water, granulated and dried to obtain a granule of each compound.

Formulation Example 5

Twenty parts of each of the present compounds (1), (3), (5), (7), (10) and (12), 3 parts of a sodium naphthalenesulfonate/formalin condensate and 75 parts of water are well pulverized and mixed. Thereafter, two parts of methyl cellulose, a thickening agent, is added to the resulting mixture and mixed to obtain a flowable concentrate of each compound.

Test examples will be shown. The present compounds are shown by Compound No. in Table 1, and compounds used as a control are shown by Compound symbol in Table 2.

Table 2

| Compound symbol | Structural formula | Remarks |
|---|---|---|
| (A) | 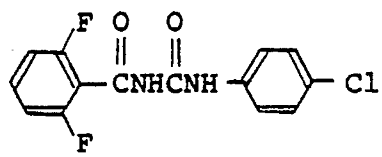 | Diflubenzuron (compound disclosed in USP No. 3,933,908). |
| (B) | 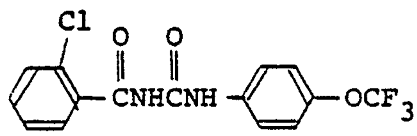 | Triflumuron (compound disclosed in USP No. 4,139,636). |
| (C) | 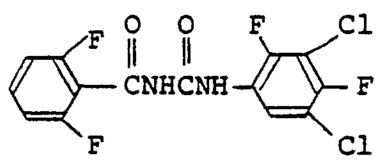 | Teflubenzuron (compound disclosed in USP No. 4,457,943). |
| (D) | 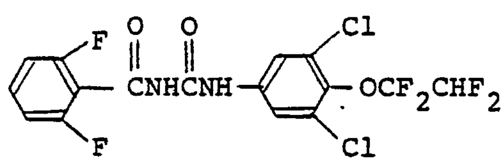 | Compound disclosed in EP No. 71,279A |
| (E) | 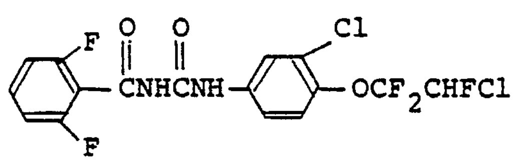 | Compound disclosed in USP No. 4,139,636. |
| (F) | 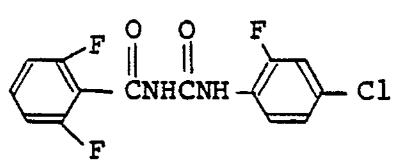 | Compound disclosed in JP-A-59-106,454. |
| (G) | 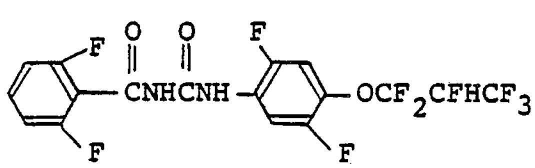 | Compound disclosed in EP No. 235,089A. |

- Cont'd -

Table 2 (Cont'd)

| | | |
|---|---|---|
| (H) | 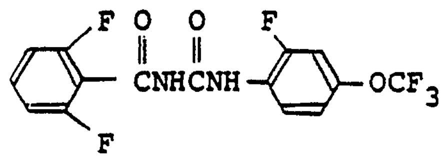 | Compound disclosed in EP No. 226,642A. |
| (I) | 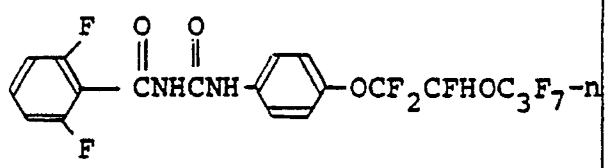 | Non-reference m.p. 175.6°C |
| (J) | 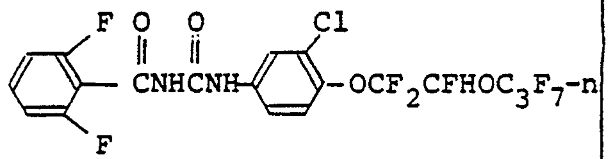 | Non-reference m.p. 138.6°C |
| (K) | 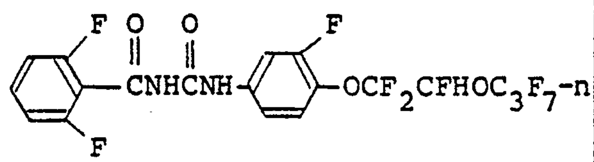 | Non-reference m.p. 176.3°C |
| (L) | 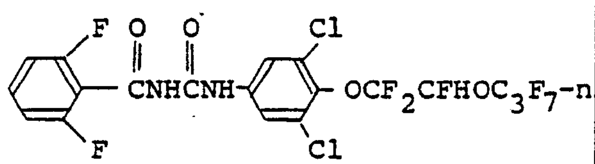 | Non-reference m.p. 118.4°C |
| (M) | 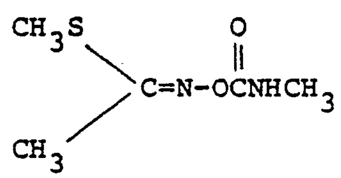 | Methomyl |

Test Example 1

The emulsifiable concentrate of each of the following present compounds prepared according to Formulation Example 1 was diluted 28,600 times with water (corresponding to 3.5 ppm). And 100 ml of each aqueous dilute emulsion thus obtained were charged in a 180-ml polyethylene cup. Then 20 last-instar larvae of common mosquito (Culex pipiens pallens) were released therein.

The larvae were bred on baits until emergence to obtain percentage of inhibition of adult emergence. The test was repeated twice.

The results are shown in Table 3.

Table 3

| Test compound | Inhibition of adult emergence (%) |
| --- | --- |
| (1) | 100 |
| (2) | 100 |
| (3) | 100 |
| (4) | 100 |
| (5) | 100 |
| (6) | 100 |
| (7) | 100 |
| (8) | 100 |
| (9) | 100 |
| (10) | 100 |
| (11) | 100 |
| (12) | 100 |
| No treatment | 10 |

Test Example 2

The emulsifiable concentrate of each of the following present compounds and controls prepared according to Formulation Example 1 was diluted 200,000 times with water (corresponding to 0.5 ppm). And 30 ml of each aqueous dilute solution thus obtained were sprayed onto cabbage cultivated in a plastic cup on a turn table, which cabbage had been planted one month before. After air-drying, 5 fourth-instar larvae of tobacco cutworm (Spodoptera litura) were released thereon, and after 5 days, the dead and alive were counted for mortality. This test was repeated three times.

The results are shown in Table 4.

Table 4

| Test compound | Mortality (%) | Test compound | Mortality (%) |
| --- | --- | --- | --- |
| (1) | 100 | (A) | 13 |
| (2) | 93 | (B) | 0 |
| (3) | 100 | (C) | 60 |
| (4) | 100 | (D) | 53 |
| (5) | 93 | (E) | 13 |
| (6) | 100 | (F) | 20 |
| (7) | 100 | (G) | 47 |
| (8) | 100 | (H) | 47 |
| (9) | 100 | (I) | 27 |
| (10) | 100 | (J) | 0 |
| (11) | 100 | (K) | 0 |
| (12) | 100 | (L) | 20 |
| | | (M) | 7 |
| | | No treatment | 0 |

Test Example 3

The emulsifiable concentrate of each of the following present compounds and controls prepared

according to Formulation Example 1 was diluted 80,000 times with water (corresponding to 1.25 ppm). Two pieces of germinated Japanese radish which had been sowed 5 or 6 days before were dipped for 30 seconds in the aqueous dilute solution. After air-drying for about 1 hour, these germinated radish were put in a cage wherein a large number of adult diamond-back moth (Plutella xylostella) (field-strain) of an age of 1-3 days after the emergence were released, and they were allowed to lay eggs. When the number of eggs per germinated radish reached 100 to 150, the radish were taken out of the cage and each two pieces thereof were put in a polyethylene cup of 5.5 cm in diameter. After 5 days, the number of hatching was counted to obtain percentage of hatching. The test was repeated twice.

The percentage of hatching was indicated in the following four grades;

A: 0%
B: 1-10%
C: 11-20%
D: 21% or more

The results are shown in Table 5.

Table 5

| Test compound | Percentage of hatching | Test compound | Percentage of hatching |
|---|---|---|---|
| (1) | A | (A) | D |
| (2) | A | (B) | D |
| (3) | A | (C) | D |
| (4) | A | (D) | D |
| (5) | A | (E) | D |
| (6) | A | (F) | D |
| (7) | A | (G) | C |
| (8) | A | (H) | C |
| (9) | A | (I) | C |
| (10) | A | (J) | D |
| (11) | A | (K) | C |
| (12) | A | (L) | D |
| | | (M) | D |
| | | No treatment | D |

## Claims

1. A benzoylurea derivative represented by the formula,

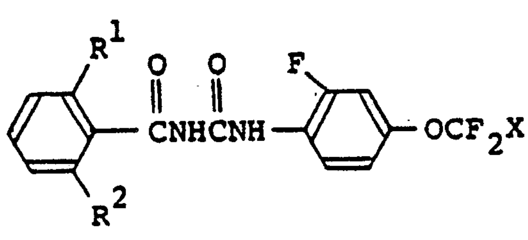

wherein $R^1$ is a fluorine or chlorine atom, $R^2$ is a fluorine atom when $R^1$ is a fluorine atom, and $R^2$ is a hydrogen atom when $R^1$ is a chlorine atom, and X is $CF_2Br$, $CCl_2H$, $CBr_2H$, CFHCl, $CFHCF_3$, $CFHOCF_3$, $CFHOC_2F_5$ or $CFHOC_3F_7$-n.

2. A benzoylurea derivative according to Claim 1, wherein X is CFHCl, $CFHCF_3$, $CFHOCF_3$ or $CFHOC_3F_7$-n.

3. A benzoylurea derivative according to Claim 1 or 2, wherein both $R^1$ and $R^2$ are a fluorine atom.

4. A benzoylurea derivative according to Claim 3, wherein both $R^1$ and $R^2$ are a fluorine atom and X is CFHCl.

5. A benzoylurea derivative according to Claim 3, wherein both $R^1$ and $R^2$ are a fluorine atom and X is $CFHCF_3$.

6. A benzoylurea derivative according to Claim 3, wherein both $R^1$ and $R^2$ are a fluorine atom and X is $CFHOCF_3$.

7. A benzoylurea derivative according to Claim 3, wherein both $R^1$ and $R^2$ are a fluorine atom and X is $CFHOC_3F_7$-n.

8. A process for producing a benzoylurea derivative according to Claim 1 which comprises allowing to react a benzoylisocyanate compound represented by the formula,

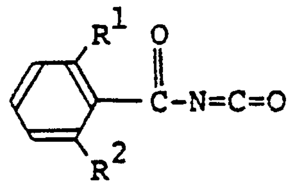

wherein $R^1$ is a fluorine or chlorine atom, and $R^2$ is a fluorine atom when $R^1$ is a fluorine atom, and $R^2$ is a hydrogen atom when $R^1$ is a chlorine atom, with an aniline compound represented by the formula,

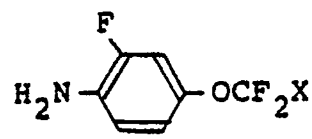

wherein X is $CF_2Br$, $CCl_2H$, $CBr_2H$, CFHCl, $CFHCF_3$, $CFHOCF_3$, $CFHOC_2F_5$ or $CFHOC_3F_7$-n.

9. A process for producing a benzoylurea derivative according to Claim 1 which comprises allowing to react a benzamide compound represented by the formula,

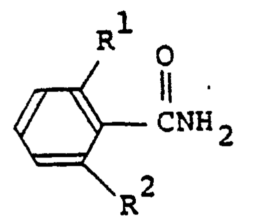

wherein $R^1$ is a fluorine or chlorine atom, and $R^2$ is a fluorine atom when $R^1$ is a fluorine atom, and $R^2$ is a hydrogen atom when $R^1$ is a chlorine atom, with an isocyanate compound represented by the formula,

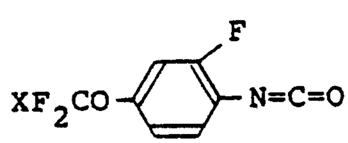

wherein X is $CF_2Br$, $CCl_2H$, $CBr_2H$, CFHCl, $CFHCF_3$, $CFHOCF_3$, $CFHOC_2F_5$ or $CFHOC_3F_7$-n.

10. An insecticidal or ovicidal composition comprising as an active ingredient a benzoylurea derivative according to Claim 1 and an adjuvant.

11. A method for controlling insects comprising administering to the insects, eggs thereof, or environment likely to contain the insects or eggs, an insecticidal composition according to Claim 10.

12. Use of an insecticidal or ovicidal composition according to Claim 10.

13. An aniline compound represented by the formula,

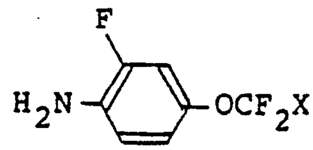

wherein X is $CF_2Br$, $CCl_2H$, $CBr_2H$, CFHCl, $CFHCF_3$, $CFHOCF_3$, $CFHOC_2F_5$ or $CFHOC_3F_7$-n.

14. A process for producing an aniline compound represented by the formula,

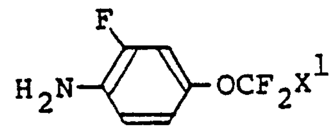

wherein $X^1$ is $CCl_2H$, $CBr_2H$, CFHCl, $CFHOCF_3$, $CFHOC_2F_5$ or $CFHOC_3F_7$-n which comprises allowing to react 3-fluoro-4-aminophenol with an olefin compound represented by the formula,

$CF_2 = Y$

wherein Y is $CCl_2$, $CBr_2$, CFCl, $CFOCF_3$, $CFOC_2F_5$ or $CFOC_3F_7$-n, in the presence of a base.

15. A process for producing an isocyanate compound represented by the formula.

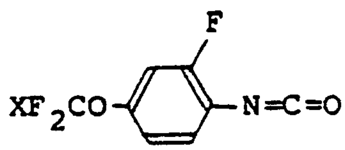

wherein X is $CF_2Br$, $CCl_2H$, $CBr_2H$, CFHCl, $CFHCF_3$, $CFHOCF_3$, $CFHOC_2F_5$ or $CFHOC_3F_7$-n, which comprises allowing to react an aniline compound represented by the formula,

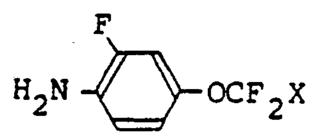

wherein X has the same meaning as defined above, with phosgene.